# EUROPÄISCHE PATENTSCHRIFT

(11) **EP 1 707 109 B1**
(45) Veröffentlichungstag und Bekanntmachung des Hinweises auf die Patenterteilung: **29.12.2010**
(21) Anmeldenummer: 06004578.8
(22) Anmeldetag: 07.03.2006
(51) Int. Cl.: A61B 5/00

(54) **Datenübertragungssystem in Verbindung mit einem Implantat**
Data transmission system in conjunction with an implant
Système de transmission de données en liaison avec un implant

(30) Priorität: 31.03.2005 DE 102005014573
(43) Veröffentlichungstag der Anmeldung: 04.10.2006
(60) Teilanmeldung: 10165798.9
(73) Patentinhaber: Stryker Trauma GmbH, 24232 Schönkirchen (DE)
(72) Erfinder: Kaiser, Edgar, Dipl.-Phys., 24253 Probsteierhagen (DE); Speiting, Andreas, Dr.-Ing., 24149 Kiel (DE)
(74) Vertreter: Maiwald Patentanwalts GmbH

(56) Entgegenhaltungen:
- WO-A-03/043688
- US-A- 4 494 950
- US-A- 5 113 859
- US-A- 5 904 708
- US-A1- 2004 158 299

## Beschreibung

Die Erfindung bezieht sich auf ein Meßsystem in Verbindung mit einem Implantat nach dem Patentanspruch 1.

Insbesondere zur Versorgung von Frakturen werden die unterschiedlichsten intramedullären und extramedullären Knochenimplantate, wie Platten, Nägel oder dergleichen eingesetzt. Generell unterscheidet man zwischen Implantaten mit transkutanem Durchgang und solchen, die ohne transkutanen Durchgang implantiert sind. Zu ersteren zählen z.B. externe Fixateure und zu letzteren Nägel oder Platten.

Es ist bekannt, physikalische und/oder chemische Meßgrößen in Verbindung mit einem Implantat zu ermitteln. So ist z.B. erwünscht, die statische und dynamische Belastung von Implantaten zu ermitteln. Aus "Journal of Biomechanics 34" (2001) S. 849-857 ist bekannt, in einem sogenannten Verriegelungs-Knochennagel eine Empfängerspule für den Empfang von externer Energie, eine Meßschaltung, einen Dehnungsmeßstreifen, eine Datenwanderschaltung und eine Sendeschaltung und - spule anzuordnen. Mit Hilfe dieser Elemente sollen die auf den Knochen einwirkenden Kräfte erfaßt werden. Aus "The Journal of Bone and joint Surgery" Volume 83-A Supplement 2, Part 1 (2001) S. 62 -65 ist bekannt, in eine Knieprothese den Dehnungsmeßstreifen einzubauen und diese über Kabel nach außen mit einem Meßgerät zu verbinden. Aus "Medical Engineering & Physics 22" (2000) S. 469 - 479 ist bekannt, z.B. bei einer Oberschenkellasche Dehnungsmeßstreifen anzubringen und diese über Kabel mit einem extrakorporalen Meßgerät zu verbinden. Aus "SPINE" Volume 25, No. 23 pp 2981 - 2986 ist die Kraftmessung an Wirbelsäulenimplantaten bekannt geworden. Aus "Sensors and Actuators" A 97-98 (2002) S. 548-556 sind Belastungsmessungen auch in Verbindung mit Dentalprothesen bekannt geworden.

Bei allen Datenerfassungen in Verbindung mit Implantaten ist die Übermittlung der Meßgrößen nach extern erforderlich. Hierzu werden z.B. Drähte verwendet, welche die Meßeinheit mit einem Meßgerät bzw. einem Meßgrößenverarbeitungsgerät verbinden. Ein solches System ist zwar u.U. für den Implantatträger wenig hinderlich, wenn das Meßgerät außen in angenehmer Weise fixiert wird, die Hindurchführung von Leitungen durch Knochen und Weichgewebe kann jedoch ständige Reizungen verursachen und sogar zu Entzündungen führen. Falls möglich und vom Aufwand her vertretbar, ist die drahtlose Telemetrie, d.h. die drahtlose Übertragung von Meßdaten nach außen als das Mittel der Wahl vorzuziehen. Eine solche drahtlose Übertragung ist etwa aus dem bereits angegebenen Artikel aus "Medical Engineering & Physics" 22 bekannt geworden. Für den Übertragungsweg werden im Nahfeld induktive oder kapazitive Kopplungen mit entsprechenden magnetischen und elektrischen Antennen bei niedrigen Frequenzen verwendet. Werden elektromagnetische Fernfeldantennen verwendet, müssen hohe Frequenzen genutzt werden. Der Übertragungsweg kann sowohl vom Datenerfassungssystem in Richtung des Implantats ("Uplink") als auch vom Implantat in Richtung des Datenerfassungssystems ("Downlink") zeigen. Die Uplink-Strecke wird häufig zur Energieversorgung des implantierten Systems durch induktive Kopplung genutzt.

Aus WO 02/094113, US 4494950 und US 2004/0158299 sind Telemetriesysteme bekannt, die auch eine akustische Datenübertragung verwenden.

Ein Nachteil bekannter Telemetriesysteme ist die starke Abschwächung, die elektromagnetische Wellen beim Durchgang durch eine metallische Abschirmung erleiden. Ein weiterer Nachteil besteht in der Schwierigkeit, die Sendeeinheit zu verwirklichen (Miniaturisierung). Besonders nachteilig wirkt sich die Abschirmung aus, wenn zur Übertragung hoher Datenraten hohe Frequenzen über 1 MHz benutzt werden. Da implantierbare Telemetriesysteme aus Gründen der Gewebeverträglichkeit und Wirtschaftlichkeit vorzugsweise in Metallkapseln aus Titan oder Implantatstahl integriert werden, gestaltet sich die Telemetrie problematisch.

Der Erfindung liegt die Aufgabe zugrunde, ein Meßsystem in Verbindung mit einem Implantat zu schaffen, das eine Übertragung von Daten problemlos ermöglicht.

Diese Aufgabe wird durch die Merkmale des Patentanspruchs 1 gelöst.

Es ist zwar denkbar, für eine Telemetrie in Verbindung mit einem Implantat die Energiequelle mit zu implantieren und auf eine externe Energieversorgung und eine externe Steuerung zu verzichten. Dies dürfte normalerweise nur eine Ausnahme sein. Typisch ist die Verwendung eines Uplinks und eines Downlinks. Der Uplink mit einer externen Sendeeinheit und einer internen intrakorporalen Empfangseinheit dient üblicherweise dazu, Steuersignale auf die Meßeinheit und auch auf den Sender für den Downlink zu übertragen. Ferner wird auf diese Weise die Energieversorgung sichergestellt, insbesondere durch induktive Kopplung. Die intrakorporale Sendeeinheit für Downlink ist z. B. vollständig metallgekapselt und kommuniziert mit einer extrakorporalen externen Empfangseinheit. Mithin liegen zwei drahtlose Übertragungsstrecken vor. Erfindungsgemäß ist zumindest die Übertragungsstrecke zwischen der internen Sendeeinheit und der externen Empfangseinheit für eine akustische Datenübermittlung ausgelegt. Die von der Meßeinheit ermittelten Daten werden im Downlink-Empfänger in akustische Daten umgewandelt bzw. zur Ansteuerung eines akustischen Wandlers verwendet zur Übertragung auf die extrakorporale Empfangseinheit. Es ist zwar denkbar, auch für die andere Übertragungsstrecke (Uplink) auf eine akustische Übertragung zurückzugreifen; dies könnte für die Steuerdaten sinnvoll sein, während für die elektrische Energieversorgung von außen die elektromagnetische Einkopplung vorzuziehen ist.

Der Vorteil des erfindungsgemäßen Meßsystems liegt darin, daß akustische Schwingungen auch metallische Abschirmungen mit geringer Dämpfung durchdringen können. Akustische Wellen können sich mit niedriger Dämpfung auch in metallischen Implantaten ausbreiten. Daher können das Implantat selbst, aber auch Knochen- und Weichteilgewebe des Implantatträgers als Übertragungsmedium für die akustische Telemetrie genutzt werden. Nach einer Ausgestaltung der Erfindung ist es daher vorteilhaft, wenn die interne Sendeeinheit akustisch an das Implantat angekoppelt ist. Bei einer intrakorporalen Anordnung des Implantats kann die externe Sendeeinheit und/oder die akustische Empfangseinheit zur Anbringung auf der Haut ausgebildet sein. So kann z.B. ein diese Teile aufnehmendes Gehäuse in geeigneter Weise an dem betreffenden Körperteil befestigt werden. Eine akustische Sendeeinheit kann auch in nicht metallischer Kapselung leicht miniaturisiert werden.

Bei einem Implanat mit transkutanem Durchgang, wie z.B. bei einem externen Fixateur, können die metallischen Stifte des Fixateurs die Übertragungsstrecke bilden und der akustische Empfänger im distalen Bereich der Stifte oder an den Anbringungspunkten angebracht werden.

Schließlich ist nach einer Ausgestaltung der Erfindung auch möglich, die Meßeinheit und die interne Sende- und Empfangseinheit an einem intramedulären Nagel anzubringen und die externe akustische Empfangseinheit lösbar am zugekehrten Ende des Nagels bzw. an einem mit dem Nagel verbindbaren Eintreibund/oder Zielinstrument anzubringen.

Vergleichsweise kann auch ein sogenannter Transponder eingesetzt werden, der implantiert wird und dessen Energieversorgung mittels induktiver und/oder kapazitiver Kopplung entlang des Uplinks erfolgt. Der Downlink, d. h. die Übertragungsstrecke vom Transponder zu einer extrakorporalen Empfangseinheit kann auf akustischem Wege erfolgen. Es ist bekannt, die Datenübertragung zu einem Transponder (Uplink) und umgekehrt (Downlink) mit der sogenannten RFID-Technik zu realisieren. Diese hat jedoch den Nachteil, daß sie leicht gestört werden kann bzw. ihrerseits Störungen verursacht, z. B. in Sicherheitssystemen. Es können z. B. Fehlalarme ausgelöst werden. Auch die Zuverlässigkeit der Datenauslesung wird durch einen akustischen Downlink erhöht, da eine elektromagnetische Übertragungsstrecke leicht gestört werden kann. Ferner können höhere Anforderungen bezüglich Datensicherheit und Persönlichkeitsschutz erfüllt werden, da der Downlink nur hergestellt werden kann, wenn der externe akustische Empfänger in direktem Kontakt mit der Haut gebracht wird. Ein Auslesen von Daten aus der Entfernung ist nicht möglich.

Der implantierbare Transponder kann eine selbständige Einheit bilden oder in ein Implantat integriert sein. Er kann in einer implantierbaren Telemetrieeinheit integriert oder mit einer solchen gekoppelt sein. Die Datenübertragung von einer externen Sendeeinheit zum implantierten Transponder kann durch elektromagnetischen Schwingungen und/oder Wellen erfolgen. Der Uplink kann jedoch ebenfalls als akustische Strecke ausgebildet sein.

Ein Transponder kann entweder nur zur Wiedergabe fest eingespeicherter Daten (read-only transponder) vorgesehen werden, z. B. um eine Artikel- oder Seriennummer eines Implantats abzufragen oder auch einen modifizierbaren Speicher enthalten, der ganz oder teilweise durch eine externe Einheit beschrieben werden kann, z. B. zur Speicherung und Wiedergabe von Patientendaten.

Die Erfindung soll nachfolgend anhand von zwei in Zeichnungen dargestellten Ausführungsbeispielen näher erläutert werden.
- Fig. 1: zeigt eine schematische Darstellung mit einem Telemetriesystem nach der Erfindung,
- Fig. 2: zeigt eine andere schematische Darstellung einer Anwendung eines Tele- metriesystems nach der Erfindung.

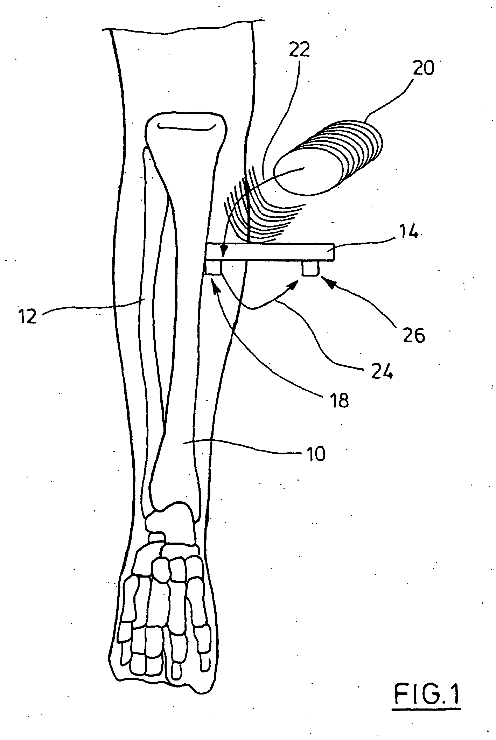

In Fig. 1 ist schematisch ein menschlicher Unterschenkel dargestellt mit Schienbein 10 und Wadenbein 12. Es ist eine Fraktur des Schienbeins 10 unterstellt, welche mit einem Fixateur extern versorgt ist. Dieser ist im einzelnen nicht dargestellt. Ersatzweise ist lediglich bei 14 ein Stift dargestellt, wie er üblicherweise bei derartigen Geräten verwendet wird. Bekanntlich wird in jedes Fraktursegment mindestens ein derartiger Stift eingeschraubt, welcher Stifte außerhalb des Beins über ein geeignetes Gestänge gegeneinander verspannt werden, um die Knochenfragmente gegeneinander zu positionieren. Da eine derartige Versorgung notorisch ist, soll hierauf im einzelnen nicht eingegangen werden.

Am Stift 14 ist nahe des Schienenbeins 10 eine Einheit 18 angebracht. Sie enthält eine elektromagnetische Empfangseinheit und eine akustische Sendeeinheit. Beide sind in einem metallischen Gehäuse gekapselt. Die akustische Sendeeinheit ist über einen geeigneten Wandler an den Stift 14 angekoppelt. Die Energie für die Einheit 18 wird elektrisch von einem elektromagnetischen Sender 20 eingekoppelt. Mithin liegt ein elektromagnetischer Uplink 22 vor.

Mit der Empfangs- und Sendeeinheit 18 ist eine nicht gezeigte Meßeinheit gekoppelt, die entweder in dem Gehäuse der Einheit 18 untergebracht oder mit dieser verbunden ist und welche gewünschte Daten physikalischer und/oder chemischer Natur erfaßt, beispielsweise Daten, welche Auskunft geben über den Heilungsverlauf der Fraktur, über die dynamische Belastung des Knochens usw. Die gemessenen Daten werden in geeigneter Weise aufbereitet, so daß sie mit Hilfe der akustischen Sendeeinheit und des akustischen Wandlers auf den Stift 14 gekoppelt werden können. Der Stift bildet mithin die akustische Übertragungsstrecke (Downlink 24). Am distalen Ende des Stiftes 14 sitzt ein akustischer Empfänger 26, der die vom akustischen Sender kommenden Daten empfängt. Sie können dann in geeigneter Weise durch einen Datenprozessor oder dergleichen aufbereitet werden.

In der Ausführungsform nach Fig. 1 ist der akustische Wandler des externen Downlinkempfängers 26 an einer Komponente außerhalb des Körpers angebracht, einem transkutanem Pin 14. Die akustische Telemetrieübertragung erfolgt ausschließlich durch das Material des Implantatsystems. Alternativ ist möglich, den akustischen Wandler des externen Downlink-Empfängers an der Körperoberfläche (Haut) anzubringen. Die akustische Telemetriebübertragung erfolgt dann durch das Implantatmaterial, Knochen- und Weichteilgewebe und durch die Haut.

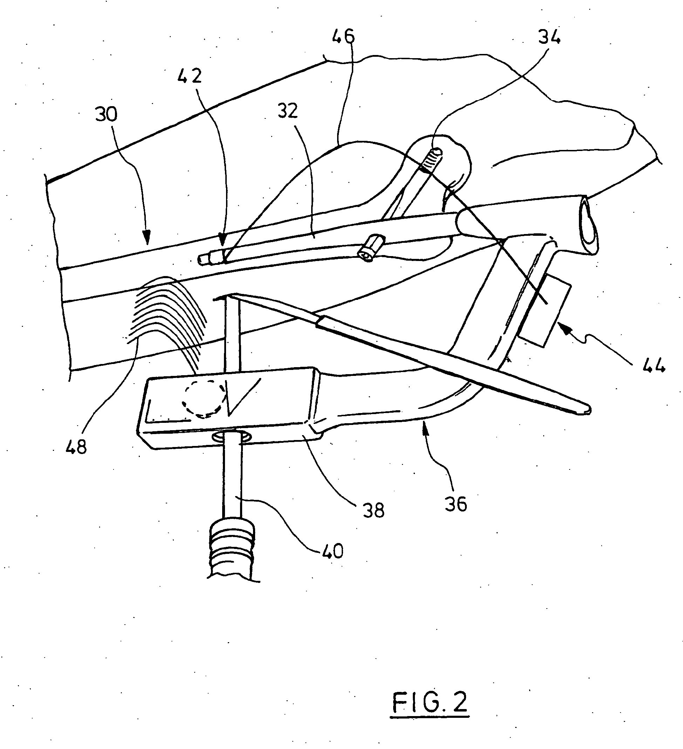

In Fig. 2 ist der Implantiervorgang eines sogenannten Gammanagels in den proximalen Femur 30 gezeigt. Der Nagel besteht aus einem Vernegelungsnagel 32 und einem schräg durch diesen gesteckten Oberschenkelhalsstift 34. Der Verriegelungsnagel 32 wird mit Hilfe eines Zielgeräts 36, das am proximalen Nagelende fest angebracht ist, eingetrieben. Es dient mithin auch als Einschlagelement. Am Zielarm 38 des Zielinstruments 36 sind Bohrungen vorgesehen zum Auffinden der Querlöcher im Nagel 32. Eine entsprechende Bohrhülse ist bei 40 dargestellt. Das beschriebene Implantiersystem ist allgemein bekannt und soll nicht weiter beschrieben werden.

Im distalen Endbereich des Nagels 32 ist eine Empfangs- und Sendeeinheit 42 angebracht, vergleichbar der Empfangs- und Sendeeinheit 18 nach Fig. 1. Sie enthält eine oder ist verbunden mit einer Meßeinheit am oder im Nagel zur Messung von Daten, die für den operierenden Arzt von Interesse sind. Am Zielinstrument 36 ist bei 44 eine akustische Empfangseinheit angebracht. Während der Operation kommuniziert der akustische Sender in der Einheit 42 mit dem akustischen Empfänger 44 über den Nagel 32 und den Zielbügel des Instruments 36. Diese Teile werden mithin als akustische Wellenleiter verwendet. Dies ist durch 46 (Downlink) angedeutet. Die Energieversorgung erfolgt über einen elektromagnetischen Uplink 48. Auch die Steuerungsdaten für die Einheit 32 bzw. die Meßeinheit können elektromagnetisch übertragen werden.

## Patentansprüche

1. Datenübertragungssystem verbindbar mit einem intramedullären Nagel (32), wobei das Datenübertragungssystem umfasst:
- eine mit dem intramedullären Nagel (32) implantierbare Messeinheit für die Messung mindestens einer physikalischen oder chemischen Messgröße am intramedullären Nagel (32) oder im Bereich von diesem,
- eine mit der Messeinheit integrierte oder koppelbare und implantierbare interne Sendeeinheit (42),
- eine extrakorporal angeordnete Empfangseinheit (44),
- wobei die internen Sende- und die extrakorporalen Empfangseinheit (44) über eine erste Übertragungsstrecke mit eine Kommunizieren können,
- eine extrakorporal angeordnete externe Sendeeinheit,
- eine von der externen Sendeeinheit ansteuerbare implantierbare Empfangseinheit (42),
- wobei die Messeinheit und die interne Sendeeinheit über eine zweite Übertragungsstrecke (48) zwischen der externen Sende- und internen Empfangseinheit angesteuert werden können,
wobei die erste Übertragungsstrecke (46) mit akustischen Schwingungen bzw. Wellen arbeitet,
**gekennzeichnet dadurch, dass** die Messeinheit und interne Sende- und Empfangseinheit an dem intramedullären Nagel (32) derart anbringbar sind, dass eine akustische Schwingungs- bzw. Wellenübertragung ermöglicht wird, und die extrakorporal angeordnete Empfangseinheit (44) lösbar am zugeordneten Ende des intramedullären Nagel (32) bzw. an einem mit dem intramedullären Nagel (32) verbundenen Eintreibinstrument (36) derart anbringbar ist, dass eine akustische Schwingungs- bzw. Wellenübertragung ermöglicht wird und der intramedulläre Nagel (32) als akustische Übertragungsstrecke dient.

2. Datenübertragungssystem nach Anspruch 1, **dadurch gekennzeichnet, dass** die zweite Übertragungsstrecke (48) mit elektromagnetischen Wellen arbeitet.

3. Datenübertragungssystem nach Anspruch 2, **dadurch gekennzeichnet, dass** über die zweite Übertragungsstrecke (48) sowohl Daten als auch Energie für den Betrieb der Messeinheit und der internen Empfangs- und Sendeeinheit (42) übertragen werden.

4. Datenübertragungssystem nach Anspruch 2 und 3, **dadurch gekennzeichnet, dass** eine dritte Übertragungssstrecke zur Übertragung von Daten mit akustischen Schwingungen bzw. Wellen arbeitet.

5. Datenübertragungssystem nach einem der Ansprüche 1 bis 4, **dadurch gekennzeichnet, dass** Messeinheit und interne Empfangs- und Sendeeinheit (42) in einem körperverträglichen Material gekapselt sind.

6. Datenübertragungssystem nach einem der Ansprüche 1 bis 5, **dadurch gekennzeichnet, dass** die interne Sendeeinheit (42) akustisch an den intramedullären Nagel (32) angekoppelt ist.

7. Datenübertragungssytem nach einem der Ansprüche 1 bis 6, **dadurch gekennzeichnet, dass** externe Sendeeinheit und/oder akustische extrakorporal angeordnete Empfangseinheit (44) zur Ankopplung auf der Haut ausgebildet sind.

8. Datenübertragungssystem nach einem der Ansprüche 1 bis 7, **dadurch gekennzeichnet, dass** bei einem intramedullären Nagel (32) mit transkutanem Durchgang mittels eines metallischen Stifts oder dergleichen dieser die erste Übertragungsstrecke bildet.

## Claims

1. A data transmission system connectable to an intramedullary nail (32), the data transmission system comprising:
- a measuring unit implantable with the intramedullary nail (32) for measuring at least one physical or chemical measuring variable at the intramedullary nail (32) or in the area thereof,
- an internal transmitter unit (42), which is integrated with or coupleable to and implantable with the measuring unit,
- an extracorporeally arranged receiver unit (44),
- wherein the internal transmitter unit and the extracorporeal receiver unit (44) being able to communicate with each other via a first transmission section,
- an extracorporeally arranged external transmitter unit,
- an implantable receiver unit (42), which is controllable by the external transmitter unit,
- wherein the measuring unit and the internal transmitter unit can be controlled via a second transmission section (48) between the external transmitter unit and the internal receiver unit,
- wherein the first transmission section (46) operates using acoustic oscillations or waves, **characterized in that** the measuring unit, the internal transmitting unit and the receiver unit are attachable to the intramedullary nail (32) so that acoustic oscillation or wave transmission is made possible, and the extracorporeally arranged receiver unit (44) is removably attachable at the corresponding end of the intramedullary nail (32) or a driving-in instrument (36), which is connected to the intramedullary nail (32), so that an acoustic oscillation or wave transmission is made possible and the intramedullary nail (32) serves as an acoustic transmission section.

2. The data transmission system according to Claim 1, **characterized in that** the second transmission section (48) operates using electromagnetic waves.

3. The data transmission system according to Claim 2, **characterized in that** both data and also energy for the operation of the measuring unit and the internal receiver and transmitter unit (42) are transmitted via the second transmission section (48).

4. The data transmission system according to Claims 2 and 3, **characterized in that** a third transmission section for transmitting data operates using acoustic oscillations or waves.

5. The data transmission system according to any one of Claims 1 to 4, **characterized in that** the measuring unit and the internal receiver and transmitter unit (42) are encapsulated in a body-compatible material.

6. The data transmission system according to any one of Claims 1 to 5, **characterized in that** the internal transmitter unit (42) is acoustically coupled to the intramedullary nail (32).

7. The data transmission system according to any one of Claims 1 to 6, **characterized in that** the external transmitter unit and/or the acoustic extracorporeally arranged receiver unit (44) are adapted to be coupled to the skin.

8. The data transmission system according to any one of Claims 1 to 7, **characterized in that**, in the case of an intramedullary nail (32) having a transcutaneous passage by means of a metal pin or the like, this pin forms the first transmission section.

## Revendications

1. Système de transmission de données pouvant être relié à une broche intramédullaire (32), le système de transmission de données comprenant :
- une unité de mesure implantable avec la broche intramédullaire (32) pour la mesure d'au moins une grandeur de mesure physique ou chimique sur la broche intramédullaire (32) ou dans la région de cette dernière,
- une unité d'émission interne (42) intégrée à l'unité de mesure ou pouvant être couplée et implantée avec cette dernière,
- une unité de réception extracorporelle (44),
- l'unité d'émission interne et l'unité de réception extracorporelle (44) pouvant communiquer entre elles par l'intermédiaire d'un premier circuit de transmission,
- une unité d'émission externe extracorporelle,
- une unité de réception (42) implantable, pouvant être contrôlée par l'unité d'émission externe,
- l'unité de mesure et l'unité d'émission interne pouvant être contrôlées par l'intermédiaire d'un deuxième circuit de transmission (48) entre l'unité d'émission externe et l'unité de réception interne,
- le premier circuit de transmission (46) fonctionnant par vibrations et/ou ondes acoustiques,
**caractérisé en ce que** l'unité de mesure et l'unité d'émission et de réception internes peuvent être montées sur la broche intramédullaire (32) de telle sorte qu'une transmission des vibrations et/ou ondes acoustiques est permise, et l'unité de réception extracorporelle (44) peut être montée de façon amovible sur l'extrémité associée de la broche intramédullaire (32) et/ou sur un instrument d'implantation (36) relié à la broche intramédullaire (32) de telle sorte qu'une transmission des vibrations et/ou ondes acoustiques est permise et que la broche intramédullaire (32) sert de circuit de transmission acoustique.

2. Système de transmission de données suivant la revendication 1, **caractérisé en ce que** le deuxième circuit de transmission (48) fonctionne par ondes électro-magnétiques.

3. Système de transmission de données suivant la revendication 2, **caractérisé en ce qu'**aussi bien des données que de l'énergie pour le fonctionnement de l'unité de mesure et de l'unité d'émission et de réception internes (42) sont transmises par l'intermédiaire du deuxième circuit de transmission (48).

4. Système de transmission de données suivant l'une des revendications 2 et 3, **caractérisé en ce qu'**un troisième circuit de transmission, destiné à la transmission de données, fonctionne par vibrations et/ou ondes acoustiques.

5. Système de transmission de données suivant l'une des revendications 1 à 4, **caractérisé en ce que** l'unité de mesure et l'unité d'émission et de réception internes (42) sont encapsulées dans un matériau compatible avec le corps.

6. Système de transmission de données suivant l'une des revendications 1 à 5, **caractérisé en ce que** l'unité d'émission interne (42) présente un couplage acoustique avec la broche intramédullaire (32).

7. Système de transmission de données suivant l'une des revendications 1 à 6, **caractérisé en ce que** l'unité d'émission externe et/ou l'unité de réception (44) extracorporelle acoustique sont réalisées pour le couplage sur la peau.

8. Système de transmission de données suivant l'une des revendications 1 à 7, **caractérisé en ce que**, pour une broche intramédullaire (32) avec passage transcutané au moyen d'une broche métallique ou analogues, cette dernière forme le premier circuit de transmission.
